# EUROPEAN PATENT APPLICATION

(11) **EP 1 101 498 A1**
(43) Date of publication of application: **23.05.2001**
(21) Application number: 99931523.7
(22) Date of filing: 23.07.1999
(51) Int. Cl.: A61K 49/00

(54) **DRUGS FOR THERAPEUTIC USE ENABLING NUCLEAR MAGNETIC RESONANCE DIAGNOSIS BY SCALAR BOND**

(30) Priority: 28.07.1998 JP 21305098
(71) Applicant: Nihon Medi-Physics Co., Ltd., Nishinomiya-shi, Hyogo 662-0918 (JP)
(72) Inventor: WASHINO, Komei, Chiyoda-ku, Tokyo 102-0073 (JP); SHIMMURA, Toshiyuki, Sodegaura-shi, Chiba 299-0266 (JP); NAKATANI, Akira, Sodegaura-shi, Chiba 299-0266 (JP); FUJIMOTO, Chieko, Sodegaura-shi, Chiba 299-0266 (JP); TANAKA, Akihiro, Sodegaura-shi, Chiba 299-0266 (JP); SERI, Shigemi, Sodegaura-shi, Chiba 299-0266 (JP); IWAI, Kumiko, Sodegaura-shi, Chiba 299-0266 (JP)
(74) Representative: Cresswell, Thomas Anthony
(86) International application number: JP9903970
(87) International publication number: WO0006207

(57) **Abstract**

Drugs for medical use characterized by containing a compound which carries in its structure at least one member selected from the group consisting of -¹⁷OH, -¹⁴NH and -³³SH, wherein the above ¹⁷O, ¹⁴N or ³³S exerts a relaxation effect on the proton bonded thereto and the relaxation effect is spreaded through the exchange of a proton in a vital component of a target organ or tissue of a living body by the above-mentioned proton, thus enabling detection by nuclear magnetic resonance. The effective circulation or distribution of such a physiologically acceptable medical drug in the target organ or tissue in vivo where it is needed can be externally detected by the nuclear magnetic resonance method before or same time as the administration of a therapeutic agent remedy to each patient.

## Description

### TECHNICAL FIELD

The present invention relates to drugs for medical use which can be detected by the nuclear magnetic resonance method, particularly to drugs for medical use which contain a compound that exerts a relaxation effect on the proton of ¹H hydrogen atom in system by the scalar coupling.

### BACKGROUND ART

It is acknowledged by everybody that medicines confer great benefits to mankind. However, medicines are not essential components for the maintenance of life and, in the case of a medicine which is a foreign material to a living body, it is not provided with a mechanism of being selectively transported to a target organ or tissue in the body unlike essential components for a living body. Consequently, the medicine administered into a living body comes to distribute itself not only to the intended site but to everywhere in the living body.

Accordingly, many medicines are not only inefficient in efficacy, but cause side effects in greater or lesser extent. Moreover, since the disease state is not uniform for each patient, the distribution state of the medicine becomes the more complicated, and it is difficult, for each patient, to select a truly effective medicine, to predict its effect and to estimate the prognosis including side effect. For example, it is not too much to say that even a dose which is generally called "normal dose" in therapeutic practice has been decided from the result of clinical trials conducted for only several hundred cases or relies upon the results accumulated by the intuition and experience of doctors. Thus, at the present state, there cannot be found no objective means for suppressing unfavorable effects resulting from unnecessary distribution of a medicine and bringing out the intended effective action thereof to the greatest extent for each patient. That is to say, the problem is that it is yet unknown how and to what extent is a medicine transported to a target organ or tissue.

The medical treatment of the present day is supported by great many peripheral sciences and technologies. Particularly marked is the progress of imaging diagnosis including X-ray diagnosis, nuclear magnetic resonance diagnosis, ultrasonic diagnosis and nuclear medicine diagnosis. Furthermore, various drugs for diagnostic use suited to each of the diagnostic methods have been being developed, and it has become possible to grasp from the outside the in vivo pharmacokinetics, such as circulation and distribution of these drugs for diagnostic use in real time. In such a case, however, though previous drugs for diagnostic use enable accurate specification of the morbid site in the living body and identification of the morbid state based on the in vivo pharmacokinetics thereof, they give virtually no suggestion, after the diagnosis, for the proper selection of therapeutic drugs to be actually used and for the prediction of their efficacy. Nevertheless, in recent years, for example, as a contrast medium for positron emission tomography (PET), one of the nuclear medicine diagnoses, 2-fluoro(¹⁸F)-2-deoxy-D-glucose, which is a glucose-analogue, has come to appear at the actual spots of medical treatment. However, though it is structurally analogous to glucose, which is an existing drug, it does not reflect the in vivo pharmacokinetics of glucose in a strict sense; further, the object of its use is limited to the diagnosis of tumors or the like, and it is not used as the means for collecting information in using glucose as a drug. Moreover, it has the disadvantage of being radioactive.

Among the several imaging diagnostic methods which enable non-invasive observation of the inside of a living body from the outside, the diagnostic method with nuclear magnetic resonance has a number of merits which are not observed in other methods of imaging diagnosis. In particular, since it gives a high contrast between soft tissues as compared with previous X-ray CT, it has a very high discriminating ability between various soft tissues such as the ones in brain, heart, liver, kidney and the like. Furthermore, it permits tomography in any desired direction and can afford blood flow information; these points are favorable in tracing in vivo behavior of drugs. Moreover, the apparatus for nuclear magnetic resonance has already been used in many medical organizations, so that the method is not restricted in its use unlike PET, of which the apparatus is provided only to limited number of medical organization.

The principle of the diagnostic method with nuclear magnetic resonance is as follows: when a high frequency pulse including the resonance frequency of the hydrogen atom ¹H is irradiated, a resonance phenomenon takes place at the hydrogen nuclei and the resonance signal is observed. This serves for imaging the state of distribution of the proton of hydrogen present as water in the living body. It is also apparent that the method is free from the risk of radiation exposure unlike the X-ray diagnostic method and nuclear medicine diagnostic method.

It has been reported that an element having a quadrupole nucleus of a nuclear spin of 1 or more, for example ¹⁷O, shortens the transverse relaxation time (T₂), which forms the basis in imaging protons, by the scalar coupling with the proton of hydrogen atom ¹H bonded thereto (S. Meiboom, J. Chem. Phys., 39, 375, 1961). Arai et al. have disclosed a method wherein, for the purpose of imaging the distribution of water H₂¹⁷O formed as a part of the metabolite of ¹⁷O₂ by using the above-mentioned technology, ¹⁷O₂ is mixed with a perfluoro compound and an emulsifier and administered into a living body (Japanese National Publication (Kohyo) 3-500896). However, Arai et al., the said inventors, have mentioned as the result of their later study that the change of signal intensity due to ¹⁷O₂ metabolysis and the condition of metabolic function do not always correspond well to each other, and a good imaging of H₂¹⁷O of the metabolite cannot be attained (JP-A-6-22936).

Further, some attempts have been made to obtain the image of proton in high sensitivity by utilizing the scalar coupling. For example, Navon et al. have developed a measuring method using H₂¹⁷O with ¹⁷O irradiation (US5479924).

### DISCLOSURE OF THE INVENTION

Overcoming the above-mentioned problems that the medical science on individual difference which forms the basis for the differentiation of patients mainly in the therapeutical aspect has not yet been established scientifically, the object of the present invention is to provide a physiologically acceptable medical drug which enables external detection of the effective circulation or distribution of the drug, used for a disease, in the target organ or tissue in vivo where it is needed by the nuclear magnetic resonance method before or same time as the administration of a therapeutic agent to each patient. Said medical drug can be used in the nuclear magnetic resonance imaging, the nuclear magnetic resonance spectrometry or the determination of relaxation time.

The present inventors have made extensive study to attain the above-mentioned object. As a result, the inventors have found that a medical drug can itself become a pharmacokinetic-diagnotic agent which can provide for each patient information on the circulation and distribution of the drug in vivo by the nuclear magnetic resonance method utilizing the scalar coupling when the drug contains, among the constituents contained in the drug, a compound which carries in its chemical structure at least one member selected from the group consisting of -OH, -NH and -SH.

Thus, the present invention provides a drug for medical use characterized by containing a compound which carries in its chemical structure at least one member selected from the group consisting of -¹⁷OH, -¹⁴NH and -³³SH, wherein the above ¹⁷O, ¹⁴N or ³³S exerts a relaxation effect on the proton of hydrogen ¹H bonded thereto and the relaxation effect is spreaded through the exchange of a proton in a vital component of a target organ or tissue in a living body with the above-mentioned proton, thus enabling detection by the nuclear magnetic resonance method.

The above-mentioned vital component of a target organ or tissue in a living body is usually water, but it may also be lactic acid, N-acetylaspartic acid, etc.

The "detection by the nuclear magnetic resonance method" signifies to measure a phenomenon wherein the ¹⁷O, ¹⁴N or ³³S in the above compound contained in the above-mentioned medical drug exerts a relaxation effect on the proton of hydrogen ¹H bonded thereto and then the proton exchanges itself with a proton in a vital component of a target organ or tissue in a living body by means of nuclear magnetic resonance imaging, nuclear magnetic resonance spectrometry or relaxation time measurement each using a proton as the detection nucleus.

According to the present invention, it becomes possible to provide a medical drug which enables external detection of the effective circulation or distribution of the drug, used for a disease, in the target organ or tissue in vivo where it is needed by the nuclear magnetic resonance method before the administration of a therapeutic agent to each patient or in real time, and it becomes further possible to establish the medical science on individual difference which forms the basis for differentiation mainly in the therapeutic aspect.

### MODE FOR CARRYING OUT THE INVENTION

The drug for medical use according to the present invention is selected from drugs which contain as a constituent a compound carrying in its chemical structure at least one member selected from the group consisting of the -OH, -NH and -SH groups. Furthermore, the whole or a part of the O, N or S atoms constituting the respective groups are substituted with their respective isotopes ¹⁷O, ¹⁴N or ³³S. Accordingly, said drug for medical use can be synthesized by using a raw material or intermediate, by which the -OH, -NH or -SH group is to be introduced, in which the whole or a part of the O, N or S atoms have been substituted with their respective stable isotopes ¹⁷O, ¹⁴N or ³³S, according to a known method for preparing a medical drug. Though ¹⁷O exists in nature only in a low concentration of 0.04%, the separation of ¹⁷O and the enrichment of ¹⁶O by ¹⁷O themselves are not the object of the present invention. With regard thereto, several methods are described in literature which include, for example, fractional distillation of heavy water, electrolysis and laser (isotope) separation.

The selection of the medical drug of the present invention containing a compound, in which the whole or a part of the O, N or S atoms of the -OH, -NH or -SH groups are substituted with their respective stable isotopes ¹⁷O, ¹⁴N or ³³S, can be made by those skilled in the art within the range not deleterious to the object of the present invention, and any desired existing medical drug can be used according to the therapeutical or diagnosis object. More specifically, selection may be made as desired according to the disease state of each patient from therapeutic agents, such as drugs for the central nervous system, drugs for the circulatory system, drugs for the digestive system, drugs for the urogenital system and drugs for tumors; nutritional or tonic agents; agents for blood and humor such as infusion; or agents for diagnosis, such as X-ray contrast media, MRI contrast media, ultrasonic contrast media and radiopharmaceuticals. Since ¹⁷O and ³³S, which are each a stable isotope element, have respectively exactly the same chemical properties as those of usual oxygen ¹⁶O and sulfur ³²S, ¹⁷O and ³³S do not show pharmacokinetics of a different nature also in a living body. The above-mentioned compound to be substituted may be any of the active ingredients of medical drugs, additives and solvents. Specifically, the active ingredient is preferably a sugar, particularly glucose, amino acid, etc; the solvent is preferably an aqueous solvent, particularly water. They may be compounded according to the formulation ratio of respective medical drugs. The dosage form may also be selected according to the respective medical drugs and may be either a solution or a lyophilized product which is dissolved before use. Further, it may have been processed with a material for a drug delivery system, such as liposome or the like.

The medical drug thus obtained is administered according to the administration routes determined for respective medical drugs. It is administered, for example, intravenously, intraarterially, intramuscularly or orally, but it may be administered precutaneously as occasion demands. When it is desired to select a proper medical drug or to estimate its efficacy, the medical drug of the present invention may be administered in advance to the full-scall administration of a medical drug. When it is desired, for example, to monitor simultaneously with the administration of a therapeutic agent whether a medical drug is properly circulated or distributed in a target organ or tissue, the medical drug of the present invention may be used as the whole or a part of the medical drug.

The dose of the medical drug of the present invention may be appropriately selected according to the uses of the drug, the degree of enrichment of ¹⁷O, ¹⁴N or ³³S, and the kind of the nuclear magnetic resonance method used as the means of determination. The nuclear magnetic resonance method used as the means of determination may be any desired one so long as it is a nuclear magnetic resonance method using a proton as the detection nucleus, but it is preferably nuclear magnetic resonance imaging, nuclear magnetic resonance spectrometry or relaxation time determination, particularly preferable being nuclear magnetic resonance imaging, which is in general use. In an experiment on the distribution of H₂¹⁷O (¹⁷O content; about 89%) using a rat cerebral ischemic model conducted by the present inventors as an experimental example which is instructive in practice, an image based on tissue infusion difference could be obtained by the T2-weighted spin echo method (nuclear magnetic resonance imaging using a proton as the detection nucleus), which is a common imaging method in medical diagnosis. Further, for example, a nuclear magnetic resonance imaging with using ¹⁷O irradiation may be more preferably used since a more enhanced sensitivity can be obtained.

For example, in the percutaneous local therapy (PEIT) of hepatic cancer, wherein pure ethanol is injected percutaneously and transhepatically into hepatic cancer to coagulate and necrotize cancer cells, there is a high possibility of the ethanol diffusing excessively to cause coagulation and necroses even of normal hepatic cells. In such a case, for example, by injecting under a nuclear magnetic resonance apparatus the present medical drug obtained by substituting the whole or a part of the -¹⁶OH group of ethanol with ¹⁷O and observing the resulting image, a therapy in which the injection range is limited only to cancer cells becomes possible. Sodium hyaluronate, which is administered into cavitas articulare in the case of osteoarthritis of knee, can be used, for example, for similar purposes.

On the other hand, in the case of infusion, which is used as the substitute of blood transfusion from the viewpoint of replenishing water, it is important how it spreads throughout a living body. In the case of an electrolyte infusion, which is of many varieties according to the kind and the concentration of the electrolyte contained therein, a proper drug must be selected for each patient to improve the disease state. In such an instance, by using the present drug in which the whole or a part of water as a solvent has been substituted with ¹⁷O, it becomes possible to judge an electrolyte infusion of what composition is to be used for improving the disease state of each patient.

### EXAMPLES

The present invention is described in detail below with reference to Examples, but the technical scope of the present invention is not limited thereby.

### Example 1 Synthesis of [3-¹⁷OH]glucose

20 g (0.07 mol) of methyl-4,6-O-benzylidene-α-D-allopyranoside was dissolved in 108 ml of pyridine to obtain a pale yellow solution. While the solution was being cooled, 43 g (0.23 mol) of p-toluenesulfonyl chloride was added thereto over 15 minutes or more, and the resulting mixture was stirred at 30°C for 48 hours. The reaction solution turned light brown and formed a white precipitate. The reaction solution was poured into ice water and extracted with chloroform. The chloroform layer was separated and washed successively with 5% sulfuric acid, 4% aqueous sodium hydrogen carbonate solution and water. Then the chloroform layer was dried over MgSO₄ and evaporated to dryness. The yellow sirupy residue was recrystallized from ethanol to obtain 32 g of a white solid. Yield 79%.

31 g (0.05 mol) of the 2,3-bis(O-p-toluenesulfonyl) derivative obtained above was dissolved in 350 ml of chloroform, and a mixture of 35 ml of a 28% sodium methoxide methanol solution and 65 ml of methanol was added thereto. The resulting mixture was kept at room temperature for 48 hours while being gently stirred and then diluted with 300 ml of water. The chloroform layer was separated, washed twice with water, then dried over MgSO₄ and evaporated to dryness. The residue obtained was crystallized from chloroform-ether to obtain a white solid.

The 500 ml of THF was added to 5.29 g (20 mmols) of methyl-2,3-anhydro-4,6-O-benzylidene-α-allopyranoside obtained above. While the resulting mixture was being stirred, 1 g of Nafion-H and 1 g of water (¹⁷O content: 10%) were slowly added thereto, and the mixture was stirred overnight at room temperature. The insolubles and Nafion-H were removed by suction filtration and the filtrate was evaporated to dryness under reduced pressure to obtain a white solid. The white solid was hydrolyzed to obtain 1.4 g of [3-¹⁷OH] glucose.

### Example 2 Preparation of agent for humor (postoperative restoring liquid) using water (¹⁷O) as solvent

The titled drug was prepared according to the formulation of "KN replenisher 4A" (a trade name, mfd. by Otsuka Pharmaceutical Factory, Inc.), which is a postoperative restoring liquid used as the replenishing agent for water and electrolytes for the whole body early after the operation. Water (¹⁶O) was added to 0.234 g of sodium chloride, 8.002 g of glucose and sodium lactate to make up the total to 100 ml. To a 0.5 ml portion thereof was added an equal quantity of water containing 10.5% of ¹⁷O so as to prepare an ¹⁷O water-containing KN replenisher 4A (¹⁷O content: 5.25%).

### Example 3 Phantom imaging of aqueous [1-¹⁷OH] glucose solution

Water was added to [1-¹⁷OH] glucose available on the market so as to prepare a solution of a concentration of 62.55 mg/ml. As a control, glucose (¹⁶O) was used to prepare a solution of a concentration of 62.9 mg/ml. These solution were respectively sealed into glass tubes and imaged with a nuclear magnetic resonance imaging apparatus of 2 tesla, Omega CSI. When the echo time was set at 200 msec, the signal intensity ratios of the aqueous [1-¹⁷OH] glucose solution and the aqueous (¹⁶O) glucose solution were respectively 79.3 and 93.1. Thus, it has become apparent that [1-¹⁷OH] glucose can be a compound which exerts a relaxation effect by the scalar coupling on the water protons of the tissue in which they are distributed.

### Example 4 Determination of relaxation time of body liquid drug (postoperative restoring liquid) using water (¹⁷O) as solvent

The relaxation time of the ¹⁷O water-containing KN replenisher 4A (¹⁷O content: 5.25%) obtained in Example 2 was determined. As the control, a KN replenisher 4A (¹⁷O content: natural abundance ratio) prepared by using water (¹⁶O) alone as the solvent was used. The relaxation time was determined by the CPMG method using a Model JNM-FSE-60 pulse NMR. As the result, the relaxation time of the ¹⁷O water-containing KN replenisher 4A was 181.19±0.50 msec, being significantly shortened as compared with the relaxation time, 626.36±1.01 msec, of the ¹⁶O water-containing replenisher 4A. From the result, it has become apparent that an effective medical drug ¹⁷O water-containing KN replenisher 4A which could exert a relaxation effect on a water proton by the scalar coupling could be prepared.

### Example 5 Determination of relaxation time of pure ethanol (¹⁷OH) in water

A 0.2 ml portion of pure ethanol (¹⁷OH, ¹⁷O content: 10%) available on the market was mixed with 0.2 ml of water (¹⁶O)(50% in terms of ethanol concentration). As a control, a solution was prepared by mixing pure ethanol (¹⁶OH) in the same manner as above. The relaxation times of the two samples thus prepared were determined by the CPMG method using a Model JNN-FSE-60 pulse NMR. As the result, the relaxation time of the pure ethanol (¹⁷OH) dilution water was 420.22±0.99 msec, being significantly shortened as compared with the relaxation time (779.12±4.91 msec) of pure ethanol (¹⁶OH) of the control. From the result, it has become apparent that pure ethanol (¹⁷OH) can become a medical drug which, after administered and when distributed, exerts a relaxation effect on the water proton of a tissue by the scalar coupling.

## Claims

1. A drug for medical use characterized by containing a compound which carries in its chemical structure at least one member selected from the group consisting of -¹⁷OH, -¹⁴NH and -³³SH, wherein the above ¹⁷O, ¹⁴N or ³³S exerts a relaxation effect on the proton bonded thereto and the relaxation effect is spreaded through the exchange of a proton in a vital component of a target organ or tissue of a living body with the above-mentioned proton, thus enabling detection by nuclear magnetic resonance.

2. The drug for medical use according to claim 1 wherein the vital component of a target organ or tissue in a living body is water, lactic acid or N-acetylaspartic acid.

3. The drug for medical use according to claim 1 or 2 wherein the drug is selected from the group consisting of therapeutic agents, nutritional or tonic agents, agents for blood and humor, and agents for diagnosis.

4. The drug for medical use according to claim 3 wherein the agent for blood and humor is infusion.

5. The drug for medical use according to claim 3 wherein the agent for diagnosis is an X-ray contrast medium, MRI contrast medium, ultrasonic contrast medium or radiophermaceutical.

6. The drug for medical use according to any one of the claims 1-5 wherein the compound carrying in its chemical structure at least one member selected from the group consisting of -¹⁷OH, -¹⁴NH and -³³SH is an active ingredient, additive or solvent of the drug for medical use.

7. The drug for medical use according to claim 6 wherein the active ingredient of the drug is a sugar.

8. The drug for medical use according to claim 7 wherein the sugar of the active ingredient of the drug is glucose.

9. The drug for medical use according to claim 6 wherein the active ingredient of the drug is an amino acid.

10. The drug for medical use according to claim 6 wherein the solvent is an aqueous solvent.

11. The drug for medical use according to any one of the claims 1-10 wherein the solvent is water.

12. The drug for medical use according to any one of the claims 1-12 wherein the drug has been processed with a material for a drug delivery system.

13. The drug for medical use according to claim 12 wherein the material for a drug delivery system is a liposome.

14. The drug for medical use according to any one of the claims 1-13 which is a compound carrying in its chemical structure at least one member selected from the group consisting of -¹⁷OH, -¹⁴NH and -³³SH and which enables the determination by means of nuclear magnetic resonance imaging, nuclear magnetic resonance spectrometry or relaxation time measurement of a phenomenon wherein the above ¹⁷O, ¹⁴N or ³³S exerts a relaxation effect on the proton bonded thereto and then the relaxation effect is spreaded through the exchange of a proton in a vital component of a target organ or tissue in a living body with the above-mentioned proton.
